# EUROPEAN PATENT SPECIFICATION

(11) **EP 3 448 184 B1**
(45) Date of publication and mention of the grant of the patent: **20.07.2022**
(21) Application number: 17715202.2
(22) Date of filing: 07.04.2017
(51) Int. Cl.: A24F 40/50, A24F 40/42, A24F 40/46, A24D 1/20

(54) **AEROSOL-GENERATING DEVICE WITH VISUAL FEEDBACK DEVICE**
AEROSOLBILDENDE VORRICHTUNG MIT VISUELLER FEEDBACKVORRICHTUNG
DISPOSITIF DE GÉNÉRATION D'AÉROSOL AVEC DISPOSITIF DE RÉTROACTION VISUEL

(30) Priority: 29.04.2016 EP 16167811
(43) Date of publication of application: 06.03.2019
(73) Proprietor: Philip Morris Products S.A., 2000 Neuchâtel (CH)
(72) Inventor: BATISTA, Rui, Nuno, 1110 Morges (CH); HEDARCHET, Stéphane, Antony, 1009 Pully (CH)
(74) Representative: Dowling, Ian
(86) International application number: PCT/EP2017/058462
(87) International publication number: WO 2017/186477

(56) References cited:
- EP-A1- 0 503 767
- EP-A2- 0 430 566
- US-A- 5 865 185
- US-A1- 2011 265 806
- US-A1- 2012 048 266
- US-A1- 2014 060 554
- US-A1- 2014 261 487

## Description

The present invention relates to an aerosol-generating device comprising a segmented visual feedback device. The invention finds particular application as a device for use in an electrically operated smoking system.

One type of aerosol-generating system is an electrically operated smoking system. Known handheld electrically operated smoking systems typically comprise an aerosol-generating device comprising a battery, control electronics and an electric heater for heating an aerosol-generating article designed specifically for use with the aerosol-generating device. In some examples, the aerosol-generating article comprises an aerosol-generating substrate, such as a tobacco rod or a tobacco plug, and the heater contained within the aerosol-generating device is inserted into or around the aerosol-generating substrate when the aerosol-generating article is inserted into the aerosol-generating device. In an alternative electrically operated smoking system, the aerosol-generating article may comprise a capsule containing an aerosol-generating substrate, such as loose tobacco.

Some electrically operated smoking systems include a simple visual feedback device to provide basic information to a consumer, such as an indication of when the device is switched on and an indication of when a heating cycle has finished.

US-2011/265806-A1 describes an electronic smoking device having a simple LED unit that indicates to a user when the device is almost empty of a liquid substrate.

US-2014/261487-A1 describes an electronic smoking device that may comprise a plurality of indicators to indicate the number of puffs taken or an estimate of the number of puffs remaining. However, since each puff may vary in flow rate and duration, such feedback is of limited use to a user.

US-5,865,185-A describes a smoking system comprising a smoking device and a replaceable tobacco flavour unit. The tobacco flavour unit may comprise an indicating means for indicating when the unit has already been heated, but it does not provide any feedback to a user during operation of the smoking system.

It would be desirable to provide an aerosol-generating device configured to provide improved feedback to a user during operation of the aerosol-generating device. It would be desirable to provide an aerosol-generating article configured to provide improved feedback to a user during use of the aerosol-generating article.

According to a first aspect of the present invention there is provided an aerosol-generating device comprising an electrical power supply, a cavity for receiving at least part of an aerosol-generating article, and at least one electrical heater positioned within the cavity. The aerosol-generating device further comprises a controller configured to control a supply of electrical power from the electrical power supply to the at least one electrical heater to activate the at least one electrical heater. The controller is configured to activate the at least one electrical heater for a total time period when at least part of an aerosol-generating article is received within the cavity. The aerosol-generating device also comprises a segmented visual feedback device, wherein a plurality of segments of the segmented visual feedback device each correspond to a different portion of the total time period. Each of the plurality of segments is configured to provide visual feedback when at least part of an aerosol-generating article is received within the cavity and when the corresponding portion of the total time period has elapsed.

Aerosol-generating devices according to the present invention are configured to heat an aerosol-generating article for a total time period. That is, the aerosol-generating device is configured to heat an aerosol-generating article until one or more aerosol-forming substrates on the aerosol-generating article having been consumed. The controller may be configured to continuously activate the at least one heater for the total time period, so that the total time period is equal to the time period during which the at least one heater is continuously activated. The controller may be configured to activate the at least one heater in a series of discrete activations until the one or more aerosol-forming substrates have been consumed. For example, the controller may be configured to activate the at least one heater only when a user is drawing on the aerosol-generating device or an aerosol-generating article received within the aerosol-generating device. In such embodiments, the total time period is equal to the sum of the time period over which the at least one heater is activated during each activation.

Advantageously, providing a segmented visual feedback device in which each of a plurality of segments is configured to provide visual feedback after a different portion of the total time period has elapsed provides a clear indication of the remaining heater activation time. That is, the segmented visual feedback device provides an accurate indication of the level of consumption of an aerosol-forming substrate on an aerosol-generating article being heated with the aerosol-generating device. This is in contrast to known devices that either provide no feedback of the level of consumption or provide only an estimate based on a number of puffs, for example.

The at least some of the different portions of the total time period may partially overlap. The different portions of the total time period may be consecutive portions of the total time period.

The segmented visual feedback device may comprise at least one segment configured to provide visual feedback at the start of the total time period. That is, the segmented visual feedback device may comprise at least one segment configured to provide visual feedback before any portion of the total time period has elapsed. Preferably, the remaining segments of the segmented visual feedback device are the plurality of segments each configured to correspond to a different portion of the total time period.

All of the segments of the visual feedback device may be the plurality of segments each configured to correspond to a different portion of the total time period.

The at least one electrical heater may comprise a plurality of electrical heaters.

The controller may be configured to simultaneously activate all of the electrical heaters each time the heaters are activated during the total time period.

The controller may be configured to sequentially activate the plurality of electrical heaters. The controller may be configured to activate and deactivate the plurality of electrical heaters one at a time. The controller may be configured to activate the plurality of electrical heaters in two or more groups, wherein all of the electrical heaters within a group are activated at the same time. The controller may be configured to activate the next heater or group of heaters after the previous heater or group of heaters has been activated but before the previous heater or group of heaters has been deactivated.

In embodiments in which the controller is configured to sequentially activate a plurality of electrical heaters, each of the plurality of segments is preferably configured to provide visual feedback when a corresponding electrical heater or group of electrical heaters has been activated for a period of time. The period of time may be the total activation time for the heater or group of electrical heaters. The period of time may be a portion of the total activation time for the electrical heater or group of electrical heaters.

Preferably, each portion of the total time period corresponds to the total activation time of a single electrical heater, such that each of the plurality of segments is configured to provide visual feedback when at least part of an aerosol-generating article is received within the cavity and when the corresponding electrical heater has been activated.

Preferably, the electrical heaters are arranged in a pattern, wherein the segments of the segmented visual feedback device are arranged in the same pattern as the electrical heaters. Advantageously, providing the electrical heaters and the segments of the segmented visual feedback device in the same pattern may further highlight the correlation between each of the plurality of segments and consumption of an aerosol-forming substrate.

The pattern of the electrical heaters and the segments of the segmented visual feedback device may include, but is not limited to, a grid of linear rows and columns, a grid of linear rows with offset columns, a two-dimensional honeycomb, one or more concentric circles, and combinations thereof.

Preferably, each of the plurality of segments is transformable from a first condition to a second condition when at least part of an aerosol-generating article is received within the cavity and when the corresponding portion of the total time period has elapsed.

The first condition may be the same for all of the plurality of segments. The first condition for at least one of the segments may be different from the first condition for the remaining segments.

The second condition may be the same for all of the plurality of segments. The second condition for at least one of the segments may be different from the second condition for the remaining segments.

The visual feedback provided by each of the plurality of segments in the second condition may vary between segments of the segmented visual feedback device so that the segmented visual feedback device displays an indicia when at least some of the plurality of segments have been transformed to the second condition. The indicia may comprise at least one of a graphical message and a text-based message for a user of the aerosol-generating device. The indicia may include a logo, such as a brand logo. The indicia may include a brand name. When all of the of the plurality of segments have been transformed to the second condition, the variation in the second condition for at least some of the segments may provide a text-based message indicating that consumption of the aerosol-forming substrate is complete.

The segmented visual feedback device may be configured to transform the plurality of segments to the second condition in a non-consecutive sequence so that the segmented visual feedback device displays an indicia when some of the segments have been transformed to the second condition and some of the segments remain in the first condition.

The segmented visual feedback device may comprise a segmented electronic display, wherein each segment of the electronic display is individually switchable, and wherein the controller is configured to switch each of the plurality of segments from the first condition to the second condition when at least part of an aerosol-generating article is received within the cavity and when the corresponding portion of the total time period has elapsed.

The segmented visual feedback device may comprise an LED array, wherein each segment comprises one or more LEDs.

The segmented visual feedback device may comprise an LCD display, wherein each segment of the segmented visual feedback device comprises one or more segments of the LCD display.

Each segment of the electronic display may be switchable between at least one of different levels of brightness and different colours. The first condition may comprise at least one of a first level of brightness or a first colour. The second condition may comprise at least one of a second level of brightness or a second colour. One of the first and second levels of brightness may be a state in which the segment is switched off.

In the first condition, all of the plurality of segments may be switched off. Each of the plurality of segments may be transformed into the second condition by switching on the segment when the corresponding portion of the total time period has elapsed.

In the first condition, all of the plurality of segments may be switched on. At least one segment of the electronic display may be transformed into the second condition by switching off the segment when the corresponding portion of the total time period has elapsed. All of the plurality of segments may be transformed into the second condition by switching off each segment when the corresponding portion of the total time period has elapsed.

At least one segment of the electronic display may be transformed into the second condition by changing the colour of the segment when the corresponding portion of the total time period has elapsed. All of the plurality of segments may be transformed into the second condition by changing the colour of each segment when the corresponding portion of the total time period has elapsed.

The controller may be configured to switch at least some of the plurality of segments into a third condition when at least part of an aerosol-generating article is received within the cavity and when the total time period has elapsed, wherein the third condition is different from the first condition and the second condition. Advantageously, switching at least some of the plurality of segments into a third condition may provide a clear indication to a user that consumption of an aerosol-forming substrate is complete. In embodiments in which each of the plurality of segments is switched off when transformed into the second condition, transforming at least some of the segments into a third condition may comprise switching on at least some of the segments. In embodiments in which each segment is switched on when transformed into the second condition, transforming at least some of the segments into a third condition may comprise switching off at least some of the segments. Transforming at least some of the plurality of segments into a third condition may comprise changing a colour of at least some of the segments. Transforming at least some of the segments into a third condition may comprise at least two of switching on at least one segment, switching off at least one segment, and changing a colour of at least one segment.

For example, all of the plurality of segments may be switched on and illuminated in a first colour when in the first condition. Each segment may be switched off when transformed into the second condition. After the total time period has elapsed, at least one of the segments may be switched on when transformed into the third condition. In the third condition, at least one of the segments may be illuminated in a second colour that is different from the first colour.

Preferably, the controller is configured to reset each segment of the electronic display to the first condition when a new aerosol-generating article is received within the cavity.

As described herein, the at least one electrical heater may comprise a plurality of electrical heaters, wherein each segment of the segmented visual feedback device provides visual feedback when a corresponding electrical heater has been activated. Each segment of the segmented visual feedback device may overlie the corresponding electrical heater so that an aerosol-generating article is positioned between the plurality of electrical heaters and the segmented visual feedback device when the aerosol-generating article is received within the cavity. Advantageously, this may further highlight the correlation between each segment of the visual feedback device and consumption of an aerosol-forming substrate.

Each of the plurality of segments may comprises a lens arranged so that a portion of an aerosol-generating article is visible through the lens when the aerosol-generating article is received within the cavity. Each lens may be transformable between a first condition and a second condition, wherein the shape of the lens in the second condition is different from the shape of the lens in the first condition. Changing the shape of the lens may change a visual appearance of an underlying portion of an aerosol-generating article when viewed through the lens, which may provide visual feedback to the user indicative of the activation of the corresponding electrical heater.

Each lens may be electronically activated. Each lens may comprise a piezoelectric actuator configured to change a shape of the lens when electrical power is supplied to the piezoelectric actuator. The controller may be configured to electronically actuate each lens to transform the lens from the first condition into the second condition when the corresponding electrical heater is activated.

Each lens may comprise a thermomechanical material configured to exhibit a change in shape when heated. Each lens may be configured so that heat from the corresponding electrical heater effects the change in shape of the lens to transform the lens into the second condition when the electrical heater is activated. Examples of suitable thermomechanical materials include thermoresponsive polymers comprising structurally modified polyvinyl alcohol. Suitable structurally modified polyvinyl alcohol includes polyvinyl alcohol that has been at least one of partially acetalised and ionised. Each lens may be formed from a thermomechanical material, such as a thermoresponsive polymer. Each lens may be formed from a conventional optical material, such as glass, and a thermoresponsive polymer coating provided on the conventional optical material.

In embodiments in which each of the plurality of segments overlies the corresponding electrical heater, each of the plurality of segments may be configured to exhibit a change in physical appearance of the segment when the segment is heated by the corresponding electrical heater. Advantageously, this provides a direct correlation between activation of each electrical heater and the transformation of the corresponding segment of the segmented visual feedback device into the second condition.

At least one segment of the plurality of segments may comprise a thermochromic material configured to exhibit a change in colour when heated by the corresponding electrical heater. Each of the plurality of segments may comprise a thermochromic material, wherein each segment is configured to change from a first colour to a second colour. One of the first and second colours may be colourless. The second colour may be the same for all of the segments. The second colour may be the same for all of the segments. The second colour for at least one of the segments may be different from the second colour for the remaining segments. The second colour for two or more of the segments may be different from the second colour for the remaining segments. Providing segments configured to exhibit different colour changes can facilitate the segmented visual feedback device displaying an indicia when at least some of the plurality of segments have been heated. The indicia may comprise at least one of a graphic, text, a logo, and a brand name. Suitable thermochromic materials include leuco dyes.

At least one segment of the plurality of segments may comprise a thermomechanical material configured to exhibit a change in shape when heated by the corresponding electrical heater. Each segment comprising a thermomechanical material may be a lens as described herein.

At least one segment of the plurality of segments may comprise a material configured to exhibit a change in at least one of a transparency of the material and a polarising effect of the material when the material is heated by the corresponding electrical heater. Changing at least one of a transparency and a polarising effect of a segment of the segmented visual feedback device may change a visual appearance of an underlying portion of an aerosol-generating article when viewed through the segment. Suitable materials include spirobenzopyran, doped oxides such as Gd(2)O(2)S:YbEr, and transparent materials comprising embedded microcrystalline silver halides.

Each electrical heater may comprise an electrically resistive material. Suitable electrically resistive materials include but are not limited to: electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal^{®} and iron-manganese-aluminium based alloys. In composite materials, the electrically resistive material may optionally be embedded in, encapsulated or coated with an insulating material or vice-versa, depending on the kinetics of energy transfer and the external physicochemical properties required.

Each electrical heater may comprise an infra-red heating element, a photonic source, or an inductive heating element.

Each electrical heater may comprise a semiconductor heater. Each semiconductor heater may comprise a substrate layer and a heating layer provided on the substrate layer. Each heating layer may be provided on a separate substrate layer. Preferably, the plurality of semiconductor heaters comprises a common substrate layer and a plurality of heating layers spaced apart from each other and each provided on the common substrate layer, wherein each heating layer forms a semiconductor heater. Advantageously, using a common substrate layer may simplify the manufacture of the plurality of semiconductor heaters and the aerosol-generating device. A suitable material for forming the substrate layer is silicon. The substrate layer may be a silicon wafer.

Each heating layer may comprise polycrystalline silicon. Each heating layer may comprise one or more dopants to provide the polycrystalline silicon with a desired electrical resistance. A suitable dopant is phosphorous. Each heating layer may be a substantially continuous layer. Each heating layer may form a pattern on the substrate layer. Advantageously, providing a heating layer that forms a pattern on the substrate layer may provide a desired temperature distribution across the semiconductor heater during operation of the heater.

The electrical power supply may comprise a direct current (DC) source. In preferred embodiments, the electrical power supply comprises a battery. The electrical power supply may comprise a Nickel-metal hydride battery, a Nickel cadmium battery, or a Lithium based battery, for example a Lithium-Cobalt, a Lithium-Iron-Phosphate or a Lithium-Polymer battery.

According to another aspect there is provided an aerosol-generating device comprising an electrical power supply, a cavity for receiving an aerosol-generating article, and a plurality of electrical heaters positioned within the cavity. The aerosol-generating device further comprises a controller configured to control a supply of electrical power from the electrical power supply to each of the electrical heaters to sequentially activate the plurality of electrical heaters. The aerosol-generating device also comprises a segmented visual feedback device, wherein each segment of the segmented visual feedback device corresponds to one of the electrical heaters. Each segment of the segmented visual feedback device is configured to provide visual feedback when an aerosol-generating article is received within the cavity and when the corresponding electrical heater has been activated.

Aerosol-generating devices according to this aspect include a segmented visual feedback device and a plurality of electrical heaters, including a direct correlation between each segment of the visual feedback device and one of the electrical heaters. Advantageously, this direct correlation provides a clear visual indication of the level of consumption of an aerosol-forming substrate on an aerosol-generating article being heated with the aerosol-generating device.

Aerosol-generating devices according to this aspect include a plurality of sequentially activated electrical heaters. Advantageously, using a plurality of sequentially activated electrical heaters to heat an aerosol-forming substrate on an aerosol-generating article may facilitate a more accurate estimation or determination of the level of consumption of the aerosol-forming substrate.

The controller is configured to sequentially activate and deactivate the plurality of electrical heaters. The controller may be configured to activate and deactivate the plurality of electrical heaters one at a time. The controller may be configured to activate the plurality of electrical heaters in two or more groups, wherein all of the electrical heaters within a group are activated at the same time. The controller may be configured to activate the next heater or group of heaters after the previous heater or group of heaters has been activated but before the previous heater or group of heaters has been deactivated.

According to another aspect there is provided an aerosol-generating article comprising a base layer, at least one aerosol-forming substrate positioned on the base layer, a cover layer overlying the at least one aerosol-forming substrate, and a segmented visual feedback device positioned on the cover layer. Each segment of the segmented visual feedback device overlies a portion of the at least one aerosol-forming substrate. Each segment of the segmented visual feedback device is configured to provide visual feedback when the corresponding portion of the at least one aerosol-forming substrate is heated.

Aerosol-generating articles comprise a segmented visual feedback device having a direct correlation between each segment of the segmented visual feedback device and a portion of the at least one aerosol-forming substrate. Advantageously, the direct correlation provides a clear and accurate visual indication of the level of consumption of the at least one aerosol-forming substrate. This is in contrast to known articles that comprise an indicating means for indicating only when the entire article has already been heated.

The at least one aerosol-forming substrate may be a single aerosol-forming substrate, wherein each segment of the segmented visual feedback device overlies a portion of the single aerosol-forming substrate.

The at least one aerosol-forming substrate may comprise a plurality of discrete aerosol-forming substrates, wherein each segment of the segmented visual feedback device overlies one of the discrete aerosol-forming substrates. Each of the plurality of discrete aerosol-forming substrates may be substantially the same. At least one of the discrete aerosol-forming substrates may be different from another of the discrete aerosol-forming substrates.

Each segment of the segmented visual feedback device may be configured to exhibit a change in physical appearance of the segment when the segment and the corresponding portion of the at least one aerosol-forming substrate are heated.

At least one segment of the segmented visual feedback device may comprise a thermochromic material configured to exhibit a change in colour when the segment and the corresponding portion of the at least one aerosol-forming substrate are heated. Each of the segments may comprise a thermochromic material, wherein each segment is configured to change from a first colour to a second colour. One of the first and second colours may be colourless. The second colour may be the same for all of the segments. The second colour for at least one of the segments may be different from the second colour for the remaining segments. The second colour for a plurality of the segments may be different from the second colour for the remaining segments. Providing segments configured to exhibit different colour changes can facilitate the segmented visual feedback device displaying an indicia when at least some of the segments have been heated. The indicia may comprise at least one of a graphic, text, a logo, and a brand name. Suitable thermochromic materials include leuco dyes.

At least one segment of the segmented visual feedback device may comprise a thermomechanical material configured to exhibit a change in shape when the segment and the corresponding portion of the at least one aerosol-forming substrate are heated. Each segment comprising a thermomechanical material may be a lens arranged so that the underlying portion of the at least one aerosol-forming substrate is visible through the lens. Preferably, each segment is configured so that, when the segment and the corresponding portion of the at least one aerosol-forming substrate are heated, the shape of the lens is changed. Changing the shape of the lens may change a visual appearance of the underlying portion of the at least one aerosol-forming substrate when viewed through the lens. Examples of suitable thermomechanical materials include thermoresponsive polymers comprising structurally modified polyvinyl alcohol. Suitable structurally modified polyvinyl alcohol includes polyvinyl alcohol that has been at least one of partially acetalised and ionised. Each lens may be formed from a thermomechanical material, such as a thermoresponsive polymer. Each lens may be formed from a conventional optical material, such as glass, and a thermoresponsive polymer coating provided on the conventional optical material.

At least one segment of the segmented visual feedback device may comprise a material configured to exhibit a change in at least one of a transparency of the material and a polarising effect of the material when the segment and the corresponding portion of the at least one aerosol-forming substrate are heated. Changing at least one of a transparency and a polarising effect of a segment of the segmented visual feedback device may change a visual appearance of the underlying portion of the at least one aerosol-forming substrate when viewed through the segment. Suitable materials include spirobenzopyran, doped oxides such as Gd(2)O(2)S:YbEr, and transparent materials comprising embedded microcrystalline silver halides.

The at least one aerosol-forming substrate may comprise a tobacco-containing material provided on the base layer. The at least one aerosol-forming substrate may comprise a solid aerosol-forming substrate. The at least one aerosol-forming substrate may comprise at least one of herb leaf, tobacco leaf, fragments of tobacco ribs, reconstituted tobacco, homogenised tobacco, extruded tobacco and expanded tobacco.

In embodiments in which the at least one aerosol-forming substrate comprises homogenised tobacco, the homogenised tobacco material may be formed by agglomerating particulate tobacco. The homogenised tobacco material may be in the form of a sheet. The homogenised tobacco material may have an aerosol-former content of greater than 5 percent on a dry weight basis. The homogenised tobacco material may have an aerosol-former content of between 5 percent and 30 percent by weight on a dry weight basis. Sheets of homogenised tobacco material may be formed by agglomerating particulate tobacco obtained by grinding or otherwise comminuting one or both of tobacco leaf lamina and tobacco leaf stems. Sheets of homogenised tobacco material may comprise one or more of tobacco dust, tobacco fines and other particulate tobacco by-products formed during, for example, the treating, handling and shipping of tobacco. Sheets of homogenised tobacco material may comprise one or more intrinsic binders, that is tobacco endogenous binders, one or more extrinsic binders, that is tobacco exogenous binders, or a combination thereof to help agglomerate the particulate tobacco. Sheets of homogenised tobacco material may comprise other additives including, but not limited to, tobacco and non-tobacco fibres, aerosol-formers, humectants, plasticisers, flavourants, fillers, aqueous and non-aqueous solvents and combinations thereof. Sheets of homogenised tobacco material are preferably formed by a casting process of the type generally comprising casting a slurry comprising particulate tobacco and one or more binders onto a conveyor belt or other support surface, drying the cast slurry to form a sheet of homogenised tobacco material and removing the sheet of homogenised tobacco material from the support surface.

The at least one aerosol-forming substrate may include at least one aerosol-former. Suitable aerosol-formers include, but are not limited to: polyhydric alcohols, such as propylene glycol, triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate

Preferred aerosol formers are polyhydric alcohols or mixtures thereof, such as propylene glycol, triethylene glycol, 1,3-butanediol and, most preferred, glycerine.

The at least one aerosol-forming substrate may comprise a single aerosol former. The at least one aerosol-forming substrate may comprise a combination of two or more aerosol formers.

In embodiments in which the at least one aerosol-forming substrate comprises a plurality of discrete aerosol-forming substrates, at least one of the discrete aerosol-forming substrates may comprise a porous carrier material and a liquid nicotine source sorbed onto the porous carrier material.

Preferably, the porous carrier material has a density of between about 0.1 grams/cubic centimetre and about 0.3 grams/cubic centimetre.

Preferably, the porous carrier material has a porosity of between about 15 percent and about 55 percent.

The porous carrier material may comprise one or more of glass, cellulose, ceramic, stainless steel, aluminium, polyethylene (PE), polypropylene, polyethylene terephthalate (PET), poly(cyclohexanedimethylene terephthalate) (PCT), polybutylene terephthalate (PBT), polytetrafluoroethylene (PTFE), expanded polytetrafluoroethylene (ePTFE), and BAREX^{®}.

Preferably, the porous carrier material is chemically inert with respect to the liquid aerosol-forming substrate.

Preferably, the liquid nicotine source comprises one or more of nicotine, nicotine base, a nicotine salt, such as nicotine-HCI, nicotine-bitartrate, or nicotine-ditartrate, or a nicotine derivative.

The nicotine source may comprise natural nicotine or synthetic nicotine.

The nicotine source may comprise pure nicotine, a solution of nicotine in an aqueous or non-aqueous solvent or a liquid tobacco extract.

The nicotine source may comprise an electrolyte forming compound. The electrolyte forming compound may be selected from the group consisting of alkali metal hydroxides, alkali metal oxides, alkali metal salts, alkaline earth metal oxides, alkaline earth metal hydroxides and combinations thereof.

The nicotine source may comprise an electrolyte forming compound selected from the group consisting of potassium hydroxide, sodium hydroxide, lithium oxide, barium oxide, potassium chloride, sodium chloride, sodium carbonate, sodium citrate, ammonium sulfate and combinations thereof.

The nicotine source may comprise an aqueous solution of nicotine, nicotine base, a nicotine salt or a nicotine derivative and an electrolyte forming compound.

The nicotine source may comprise other components including, but not limited to, natural flavours, artificial flavours and antioxidants.

At least one of the discrete aerosol-forming substrates may comprise the porous carrier material and the nicotine source sorbed onto the porous carrier material, and at least one of the discrete aerosol-forming substrates may comprise a porous carrier material and an acid source sorbed onto the porous carrier material. During use, volatile compounds from the nicotine source and the acid source may react in the gas phase to form an aerosol comprising nicotine salt particles.

The acid source may comprise an organic acid or an inorganic acid. Preferably, the acid source comprises an organic acid, more preferably a carboxylic acid, most preferably an alphaketo or 2-oxo acid or lactic acid.

Preferably, the acid comprises an acid selected from the group consisting of 3-methyl-2-oxopentanoic acid, pyruvic acid, 2-oxopentanoic acid, 4-methyl-2-oxopentanoic acid, 3-methyl-2-oxobutanoic acid, 2-oxooctanoic acid, lactic acid and combinations thereof. Advantageously, the acid comprises pyruvic acid or lactic acid. More advantageously, the acid comprises lactic acid.

The invention is further described, by way of example only, with reference to the accompanying drawings in which:
Figure 1 shows a cross-sectional view of an aerosol-generating device according to an embodiment of the present invention;
Figure 2 shows a top view of the plurality of electrical heaters of the aerosol-generating device of Figure 1;
Figure 3 shows a top view of the segmented visual feedback device of the aerosol-generating device of Figure 1;
Figure 4 shows a perspective view of a first embodiment of an aerosol-generating article for use with the aerosol-generating device of Figure 1;
Figure 5 shows a perspective view of a second embodiment of an aerosol-generating article for use with the aerosol-generating device of Figure 1;
Figure 6 shows a cross-sectional view of the aerosol-generating article of Figure 5 combined with the aerosol-generating device of Figure 1 to form an aerosol-generating system;
Figure 7 shows a perspective exploded view of an aerosol-generating article; and
Figure 8 shows a cross-sectional view of an aerosol-generating device for use with the aerosol-generating article of Figure 7.

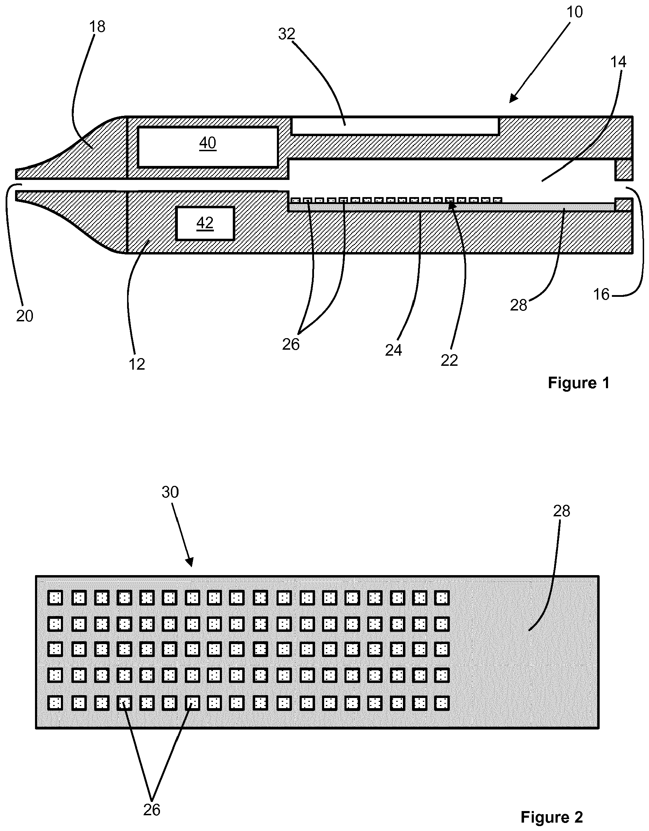

Figure 1 shows a cross-sectional view of an aerosol-generating device 10 according to an embodiment of the present invention. The aerosol-generating device 10 comprises a housing 12 defining a cavity 14 for receiving an aerosol-generating article. An air inlet 16 is provided at an upstream end of the cavity 14 and a mouthpiece 18 is provided at a downstream end of the housing 12. An air outlet 20 is provided in the mouthpiece 18 in fluid communication with the cavity 14 so that an airflow path is defined through the cavity 14 between the air inlet 16 and the air outlet 20. During use, a user draws on the mouthpiece 18 to draw air into the cavity 14 through the air inlet 16 and out of the cavity 14 through the air outlet 20.

The aerosol-generating device 10 further comprises a plurality of electrical heaters 22 provided on a planar wall 24 of the cavity 14. Each of the electrical heaters 22 comprises a heater element 26 provided on a common support layer 28. The plurality of electrical heaters 22 form a heater array 30, which is shown more clearly in Figure 2.

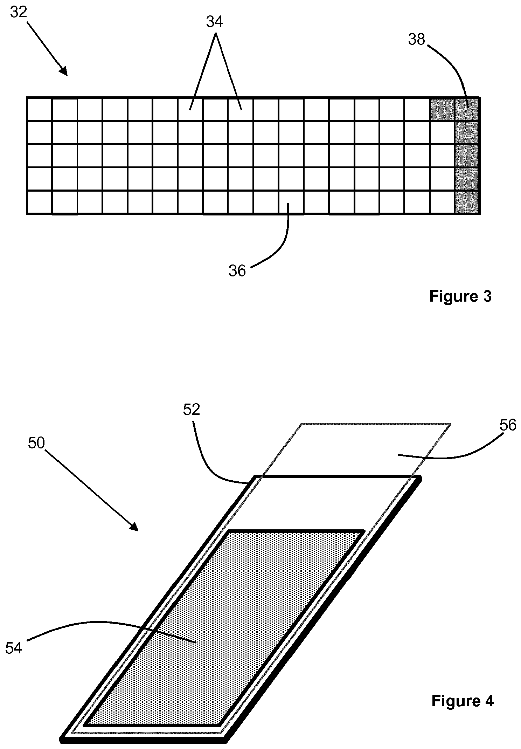

The aerosol-generating device also comprises a segmented visual feedback device 32, which is shown more clearly in Figure 3. The segmented visual feedback device 32 comprises a plurality of segments 34 arranged in an array having the same pattern as the heater array 30. In the embodiment shown in Figures 1 to 3, each segment 34 comprises a blue LED, a red LED and a green LED so that each segment 34 can be illuminated in a variety of different colours.

The aerosol-generating device 10 further comprises an electrical power supply 40 and a controller 42 positioned within the housing 12. When an aerosol-generating article is received within the cavity 14, the controller 42 controls a supply of electrical current from the electrical power supply 40 to each electrical heater 22 to activate the electrical heater 22. The controller 42 is configured to activate the plurality of electrical heaters 22 in groups, with each group being activated and deactivated sequentially. The controller 42 is also configured to switch each of the segments 34 of the segmented visual feedback device 32 from a first condition 36 to a second condition 38 when the corresponding electrical heater 22 in the heater array 30 is activated. In the first condition 36 each segment 34 is switched off, and in the second condition 38 each segment 34 is switched on an illuminated in a first colour. When all of the electrical heaters 22 have been activated, the controller 42 is configured to switch all of the segments 34 of the segmented visual feedback device 32 to a third condition. In the third condition all of the segments 34 are switched on and illuminated in a second colour that is different from the first colour. When the aerosol-generating article is removed from the cavity 14 and a new aerosol-generating article is inserted into the cavity 14, the controller 42 resets all of the segments 34 to the first condition.

Figure 4 shows a first aerosol-generating article 50 for use with the aerosol-generating device 10 of Figures 1 to 3. The aerosol-generating article 50 comprises a base layer 52 and an aerosol-forming substrate 54 provided on the base layer 52. The aerosol-forming substrate 54 comprises a substantially continuous layer of a solid tobacco-containing material. A removable cover layer 56 is secured to the base layer 52 to seal the aerosol-forming substrate 54 between the base layer 52 and the removable cover layer 56. The removable cover layer is formed from a non-porous polymeric film.

During use, the removable cover layer 56 is removed from the base layer 52 and the aerosol-generating article 50 is inserted into the cavity 14 of the aerosol-generating device 10 shown in Figure 1 to form an aerosol-generating system. The controller 42 then sequentially activates and deactivates groups of the electrical heaters 22 to sequentially heat discrete portions of the aerosol-forming substrate 54. Each time an electrical heater 22 is activated, the controller 42 switches the corresponding segment 34 of the segmented visual feedback device 32 into the second condition to indicate that the corresponding portion of the aerosol-forming substrate 54 has been consumed. In this way, the segmented visual feedback device 32 provides a clear indication of the level of consumption of the aerosol-forming substrate 54.

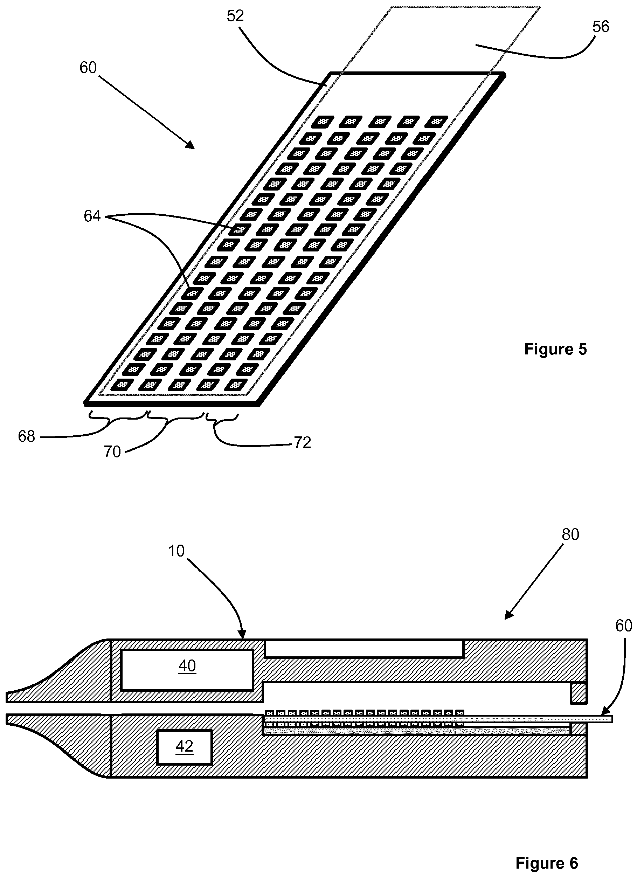

Figure 5 shows a second aerosol-generating article 60 for use with the aerosol-generating device 10 of Figures 1 to 3. The aerosol-generating article 60 comprises a base layer 52 and a cover layer 56 identical to the base layer 52 and the cover layer 56 of the aerosol-generating article 50 shown in Figure 4. However, the aerosol-generating article 60 comprises a plurality of discrete aerosol-forming substrates 64 positioned on the base layer 52 and sealed between the base layer 52 and the cover layer 56. Each of the aerosol-forming substrates 64 comprises a porous substrate material and a liquid aerosol-forming substrate sorbed onto the porous substrate material.

The plurality of aerosol-forming substrates 64 is divided into three groups: a plurality of first aerosol-forming substrates 68 each comprising a liquid nicotine solution; a plurality of second aerosol-forming substrates 70 each comprising a volatile acid; and a plurality of third aerosol-forming substrates 72 each comprising a flavourant.

During use, the removable cover layer 56 is removed from the base layer 52 and the aerosol-generating article 60 is inserted into the cavity 14 of the aerosol-generating device 10 shown in Figure 1 to form an aerosol-generating system 80, as shown in Figure 6. The arrangement of the aerosol-forming substrates 64 is such that each aerosol-forming substrate 64 overlies an electrical heater 22 when the aerosol-generating article 60 is received within the cavity 14.

The controller 42 then sequentially activates and deactivates groups of the electrical heaters 22 to sequentially heat the discrete aerosol-forming substrates 64. At each stage of the sequential activation, the controller 42 activates the appropriate electrical heaters 22 to simultaneously heat one of the first aerosol-forming substrates 68, one of the second aerosol-forming substrates 70 and one of the third aerosol-forming substrates 72. The nicotine vapour released from the heated first aerosol-forming substrate 68 and the acid vapour released from the heated second aerosol-forming substrate 70 react in the gas phase to form an aerosol comprising nicotine salt particles for delivery to the user through the air outlet 20. The flavourant released from the heated third aerosol-forming substrate 72 imparts a flavour to the aerosol delivered to the user.

Each time an electrical heater 22 is activated, the controller 42 switches the corresponding segment 34 of the segmented visual feedback device 32 into the second condition to indicate that the corresponding discrete aerosol-forming substrate 64 has been consumed. In this way, the segmented visual feedback device 32 provides a clear indication of the level of consumption of the plurality of discrete aerosol-forming substrates 64.

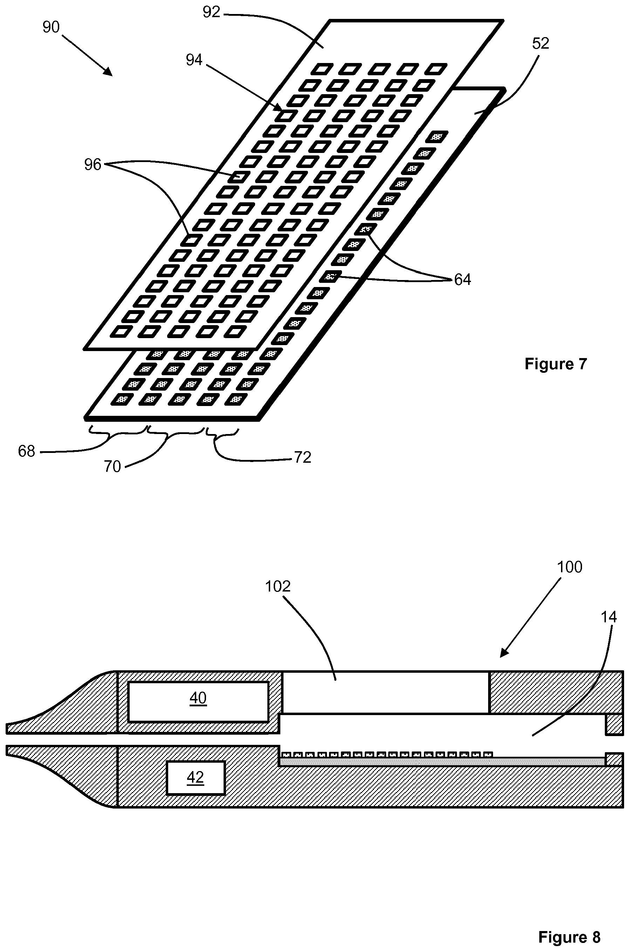

Figure 7 shows an exploded view of an aerosol-generating article 90. The aerosol-generating article 90 comprises a base layer 52 and a plurality of discrete aerosol-forming substrates 64 that are identical to the base layer 52 and aerosol-forming substrates 64 of the aerosol-generating article 60 shown in Figure 5. Therefore, like reference numerals are used to designate like parts.

The aerosol-generating article 90 comprises a cover layer 92 attached to the base layer 52 and overlying the plurality of discrete aerosol-forming substrates 64. A segmented visual feedback device 94 is positioned on the cover layer 92, the segmented visual feedback device 94 comprising a plurality of segments 96. The pattern formed by the plurality of segments 96 is identical to the pattern formed by the plurality of discrete aerosol-forming substrates 64 so that each segment 96 overlies a discrete aerosol-forming substrate 64.

Each of the segments 96 of the segmented visual feedback device 94 comprises a material that exhibits a change in the appearance of the material when heated. For example, each segment 96 may comprise at least one of a thermomechanical material, a thermochromic material, a material configured to exhibit a change in transparency when heater, and a material configured to exhibit a change in a polarising effect of the material when heated.

Figure 8 shows a cross-sectional view of an aerosol-generating device 100 for use with the aerosol-generating article 90 of Figure 7. The aerosol-generating article 100 is substantially the same in construction and operation as the aerosol-generating article 10 of Figures 1 to 3, and like reference numerals designate like parts. The aerosol-generating article 100 comprises a transparent window 102 instead of a segmented visual feedback device. The transparent window 102 is positioned so that it overlies the segmented visual feedback device 94 of the aerosol-generating article 90 when the aerosol-generating article 90 is received within the cavity 14. When the aerosol-generating article 90 is received within the cavity 14, the controller 42 sequentially activates the electrical heaters 22 to sequentially heat the plurality of discrete aerosol-forming substrates 64, as described with reference to Figure 6. When each of the discrete aerosol-forming substrates 64 is heated, the heat causes the material of the corresponding segment 96 of the segmented visual feedback device 94 to exhibit a change in appearance. The change in appearance of each segment 96 can be observed through the transparent window 102, and in this way the segmented visual feedback device 94 provides a clear indication of the level of consumption of the plurality of discrete aerosol-forming substrates 64.

## Claims

1. An aerosol-generating device (10) comprising:
an electrical power supply (40);
a cavity (14) for receiving at least part of an aerosol-generating article (50);
at least one electrical heater (22) positioned within the cavity;
a controller (42) configured to control a supply of electrical power from the electrical power supply to the at least one electrical heater to activate the at least one electrical heater, wherein the controller is configured to activate the at least one electrical heater for a total time period when at least part of an aerosol-generating article is received within the cavity; and
a segmented visual feedback device (32), wherein a plurality of segments (34) of the segmented visual feedback device each correspond to a different portion of the total time period, and wherein each of the plurality of segments is configured to provide visual feedback when at least part of an aerosol-generating article is received within the cavity and when the corresponding portion of the total time period has elapsed.

2. An aerosol-generating device according to claim 1, wherein the at least one electrical heater comprises a plurality of electrical heaters.

3. An aerosol-generating device according to claim 2, wherein the controller is configured to sequentially activate the plurality of electrical heaters.

4. An aerosol-generating device according to claim 3, wherein each portion of the total time period corresponds to the total activation time of a single electrical heater, and wherein each of the plurality of segments is configured to provide visual feedback when at least part of an aerosol-generating article is received within the cavity and when the corresponding electrical heater has been activated.

5. An aerosol-generating device according to claim 4, wherein the electrical heaters are arranged in a pattern, and wherein the segments of the segmented visual feedback device are arranged in the same pattern as the electrical heaters.

6. An aerosol-generating device according to any preceding claim, wherein each of the plurality of segments is transformable from a first condition to a second condition when at least part of an aerosol-generating article is received within the cavity and when the corresponding portion of the total time period has elapsed.

7. An aerosol-generating device according to claim 6, wherein the visual feedback provided by each of the plurality of segments in the second condition varies between segments of the segmented visual feedback device so that the segmented visual feedback device displays an indicia when at least some of the plurality of segments have been switched to the second condition.

8. An aerosol-generating device according to claim 6 or 7, wherein the segmented visual feedback device is configured to transform the plurality of segments to the second condition in a non-consecutive sequence so that the segmented visual feedback device displays an indicia when some of the plurality of segments have been transformed to the second condition and some of the segments remain in the first condition.

9. An aerosol-generating device according to claim 6, 7 or 8, wherein the segmented visual feedback device comprises a segmented electronic display, wherein each segment of the electronic display is individually switchable, and wherein the controller is configured to switch each of the plurality of segments from the first condition to the second condition when at least part of an aerosol-generating article is received within the cavity and when the corresponding portion of the total time period has elapsed.

10. An aerosol-generating device according to claim 9, wherein each segment of the electronic display is switchable between at least one of different levels of brightness and different colours.

11. An aerosol-generating device according to claim 9 or 10, wherein the controller is configured to switch at least some of the segments of the electronic display into a third condition when at least part of an aerosol-generating article is received within the cavity and when the total time period has elapsed, wherein the third condition is different to the first condition and the second condition.

12. An aerosol-generating device according to claim 9, 10 or 11, wherein the controller is configured to reset each of the plurality of segments to the first condition when at least part of a new aerosol-generating article is received within the cavity.

## Patentansprüche

1. Aerosolerzeugungsvorrichtung (10), aufweisend:
eine elektrische Energieversorgung (40);
einen Hohlraum (14) zur Aufnahme wenigstens eines Teils des aerosolerzeugenden Artikels (50);
wenigstens eine, innerhalb des Hohlraums positionierte elektrische Heizvorrichtung (22);
einen Regler (42), der zur Regelung einer Zufuhr von elektrischer Energie von der elektrischen Energieversorgung an die wenigstens eine elektrische Heizvorrichtung zur Aktivierung der wenigstens einen elektrischen Heizvorrichtung ausgelegt ist, wobei der Regler zur Aktivierung der wenigstens einen elektrischen Heizvorrichtung für eine Gesamtzeit ausgelegt ist, wenn wenigstens ein Teil eines aerosolerzeugenden Artikels innerhalb des Hohlraums aufgenommen ist; und
eine segmentierte visuelle Rückmeldungsvorrichtung (32), wobei eine Vielzahl von Segmenten (34) der segmentierten visuellen Rückmeldungsvorrichtung jeweils einem unterschiedlichen Abschnitt der Gesamtzeit entspricht, und wobei jedes der Vielzahl von Segmenten zum Vorsehen einer visuellen Rückmeldung ausgelegt ist, wenn wenigstens ein Teil eines aerosolerzeugenden Artikels innerhalb des Hohlraums aufgenommen ist und wenn der entsprechende Abschnitt der Gesamtzeit verstrichen ist.

2. Aerosolerzeugungsvorrichtung nach Anspruch 1, wobei die wenigstens eine elektrische Heizvorrichtung eine Vielzahl von elektrischen Heizvorrichtungen umfasst.

3. Aerosolerzeugungsvorrichtung nach Anspruch 2, wobei der Regler zur sequenziellen Aktivierung der Vielzahl von elektrischen Heizvorrichtungen ausgelegt ist.

4. Aerosolerzeugungsvorrichtung nach Anspruch 3, wobei jeder Abschnitt der Gesamtzeit der Gesamtzeit der Aktivierung einer einzelnen elektrischen Heizvorrichtung entspricht, und wobei jedes der Vielzahl von Segmenten zum Vorsehen einer visuellen Rückmeldung ausgelegt ist, wenn wenigstens ein Teil eines aerosolerzeugenden Artikels innerhalb des Hohlraums aufgenommen ist und wenn die entsprechende elektrische Heizvorrichtung aktiviert worden ist.

5. Aerosolerzeugungsvorrichtung nach Anspruch 4, wobei die elektrischen Heizvorrichtungen in einem Muster angeordnet sind, und wobei die Segmente der segmentierten visuellen Rückmeldungsvorrichtung in dem gleichen Muster wie die elektrischen Heizvorrichtungen angeordnet sind.

6. Aerosolerzeugungsvorrichtung nach einem beliebigen vorhergehenden Anspruch, wobei jedes der Vielzahl von Segmenten von einem ersten Zustand in einen zweiten Zustand umwandelbar ist, wenn wenigstens ein Teil eines aerosolerzeugenden Artikels innerhalb des Hohlraums aufgenommen ist und wenn der entsprechende Abschnitt der Gesamtzeit verstrichen ist.

7. Aerosolerzeugungsvorrichtung nach Anspruch 6, wobei die visuelle Rückmeldung, die durch jedes der Vielzahl von Segmenten in dem zweiten Zustand vorgesehen ist, zwischen Segmenten der segmentierten visuellen Rückmeldungsvorrichtung variiert, sodass die segmentierte visuelle Rückmeldungsvorrichtung ein Zeichen anzeigt, wenn zumindest einige der Vielzahl von Segmenten in den zweiten Zustand geschaltet worden sind.

8. Aerosolerzeugungsvorrichtung nach Anspruch 6 oder 7, wobei die segmentierte visuelle Rückmeldungsvorrichtung zur Umwandlung der Vielzahl von Segmenten in einer nicht aufeinanderfolgenden Sequenz in den zweiten Zustand ausgelegt ist, sodass die segmentierte visuelle Rückmeldungsvorrichtung ein Zeichen anzeigt, wenn einige der Vielzahl von Segmenten in den zweiten Zustand umgewandelt wurden und einige der Segmente in dem ersten Zustand verbleiben.

9. Aerosolerzeugungsvorrichtung nach Anspruch 6, 7 oder 8, wobei die segmentierte visuelle Rückmeldungsvorrichtung eine segmentierte elektronische Anzeige umfasst, wobei jedes Segment der elektronischen Anzeige einzeln umschaltbar ist, und wobei der Regler zum Umschalten jedes der Vielzahl von Segmenten von dem ersten Zustand in den zweiten Zustand ausgelegt ist, wenn wenigstens ein Teil eines aerosolerzeugenden Artikels innerhalb des Hohlraums aufgenommen ist und wenn der entsprechende Abschnitt der Gesamtzeit verstrichen ist.

10. Aerosolerzeugungsvorrichtung nach Anspruch 9, wobei jedes Segment der elektronischen Anzeige zwischen wenigstens einem von verschiedenen Helligkeitsniveaus und verschiedenen Farben umschaltbar ist.

11. Aerosolerzeugungsvorrichtung nach Anspruch 9 oder 10, wobei der Regler zum Umschalten zumindest einiger der Segmente der elektronischen Anzeige in einen dritten Zustand ausgelegt ist, wenn wenigstens ein Teil eines aerosolerzeugenden Artikels innerhalb des Hohlraums aufgenommen ist und wenn die Gesamtzeit verstrichen ist, wobei der dritte Zustand von dem ersten Zustand und dem zweiten Zustand verschieden ist.

12. Aerosolerzeugungsvorrichtung nach Anspruch 9, 10 oder 11, wobei der Regler zum Zurücksetzen jedes der Vielzahl von Segmenten in den ersten Zustand ausgelegt ist, wenn wenigstens ein Teil eines neuen aerosolerzeugenden Artikels innerhalb des Hohlraums aufgenommen ist.

## Revendications

1. Dispositif de génération d'aérosol (10) comprenant :
une alimentation électrique (40) ;
une cavité (14) pour la réception d'au moins une partie d'un article de génération d'aérosol (50) ;
au moins un dispositif de chauffage électrique (22) positionné à l'intérieur de la cavité ;
un dispositif de commande (42) configuré pour commander une alimentation en énergie électrique à partir de l'alimentation électrique de l'au moins un dispositif de chauffage électrique pour activer l'au moins un dispositif de chauffage électrique, dans lequel le dispositif de commande est configuré pour activer l'au moins un dispositif de chauffage électrique pendant une période totale lorsqu'au moins une partie d'un article de génération d'aérosol est reçue à l'intérieur de la cavité ; et
un dispositif de rétroaction visuelle segmenté (32), dans lequel une pluralité de segments (34) du dispositif de rétroaction visuelle segmenté correspondent chacun à une portion différente de la période totale, et dans lequel chacun de la pluralité de segments est configuré pour fournir une rétroaction visuelle lorsqu'au moins une partie d'un article de génération d'aérosol est reçue à l'intérieur de la cavité et lorsque la portion correspondante de la période totale s'est écoulée.

2. Dispositif de génération d'aérosol selon la revendication 1, dans lequel l'au moins un dispositif de chauffage électrique comprend une pluralité de dispositifs de chauffage électriques.

3. Dispositif de génération d'aérosol selon la revendication 2, dans lequel le dispositif de commande est configuré pour activer séquentiellement la pluralité de dispositifs de chauffage électriques.

4. Dispositif de génération d'aérosol selon la revendication 3, dans lequel chaque portion de la période totale correspond au temps d'activation total d'un seul dispositif de chauffage électrique, et dans lequel chacun de la pluralité de segments est configuré pour fournir une rétroaction visuelle lorsqu'au moins une partie d'un article de génération d'aérosol est reçue à l'intérieur de la cavité et lorsque le dispositif de chauffage électrique correspondant a été activé.

5. Dispositif de génération d'aérosol selon la revendication 4, dans lequel les dispositifs de chauffage électriques sont agencés selon un motif, et dans lequel les segments du dispositif de rétroaction visuelle segmenté sont agencés selon le même motif que les dispositifs de chauffage électriques.

6. Dispositif de génération d'aérosol selon l'une quelconque des revendications précédentes, dans lequel chacun de la pluralité de segments peut être transformé d'une première condition à une deuxième condition lorsqu'au moins une partie d'un article de génération d'aérosol est reçue à l'intérieur de la cavité et lorsque la portion correspondante de la période totale s'est écoulée.

7. Dispositif de génération d'aérosol selon la revendication 6, dans lequel la rétroaction visuelle fournie par chacun de la pluralité de segments dans la deuxième condition varie entre des segments du dispositif de rétroaction visuelle segmenté de sorte que le dispositif de rétroaction visuelle segmenté affiche un indice lorsqu'au moins certains de la pluralité de segments ont été commutés vers la deuxième condition.

8. Dispositif de génération d'aérosol selon la revendication 6 ou 7, dans lequel le dispositif de rétroaction visuelle segmenté est configuré pour transformer la pluralité de segments en la deuxième condition en une séquence non consécutive de sorte que le dispositif de rétroaction visuelle segmenté affiche un indice lorsque certains de la pluralité de segments ont été transformés en la deuxième condition et que certains des segments restent dans la première condition.

9. Dispositif de génération d'aérosol selon la revendication 6, 7 ou 8, dans lequel le dispositif de rétroaction visuelle segmenté comprend un affichage électronique segmenté, dans lequel chaque segment de l'affichage électronique est commutable individuellement, et dans lequel le dispositif de commande est configuré pour commuter chacun de la pluralité de segments de la première condition à la deuxième condition lorsqu'au moins une partie d'un article de génération d'aérosol est reçue à l'intérieur de la cavité et lorsque la portion correspondante de la période totale s'est écoulée.

10. Dispositif de génération d'aérosol selon la revendication 9, dans lequel chaque segment de l'affichage électronique peut être commuté entre au moins l'un parmi différents niveaux de luminosité et différentes couleurs.

11. Dispositif de génération d'aérosol selon la revendication 9 ou 10, dans lequel le dispositif de commande est configuré pour commuter au moins certains des segments de l'affichage électronique dans une troisième condition lorsqu'au moins une partie d'un article de génération d'aérosol est reçue à l'intérieur de la cavité et lorsque la période totale s'est écoulée, dans lequel la troisième condition est différente de la première condition et de la deuxième condition.

12. Dispositif de génération d'aérosol selon la revendication 9, 10 ou 11, dans lequel le dispositif de commande est configuré pour rétablir chacun de la pluralité de segments dans la première condition lorsqu'au moins une partie d'un nouvel article de génération d'aérosol est reçue à l'intérieur de la cavité.
